# EUROPEAN PATENT APPLICATION

(11) **EP 4 772 175 A1**
(43) Date of publication of application: **08.07.2026**
(21) Application number: 24859819.5
(22) Date of filing: 28.08.2024
(51) Int. Cl.: A61K 9/70, A61K 47/32, A61K 47/36, A61K 47/38, A61P 1/02

(54) **FILM PREPARATION HAVING ADHESIVENESS TO INSIDE OF ANIMAL ORAL CAVITY**

(30) Priority: 29.08.2023 JP 2023139309
(71) Applicant: Petina Co., Ltd., Tokyo 107-0052 (JP); Kyukyu Pharmaceutical Co., Ltd., Tokyo 103-0023 (JP)
(72) Inventor: IRIYAMA, Kosei, Chiba-shi, Chiba 260-0024 (JP); AWAMURA, Tsutomu, Imizu-shi, Toyama 939-0351 (JP); KOKAJI, Kazuhiko, Imizu-shi, Toyama 939-0351 (JP)
(74) Representative: Plougmann Vingtoft a/s
(86) International application number: PCT/JP2024/030670
(87) International publication number: WO 2025/047788

(57) **Abstract**

The present invention aims to provide a veterinary formulation that can retain a drug in the oral cavity of an animal for a sufficient time period or can quickly release the drug in the oral cavity of the animal. The present invention relates to an animal oral cavity-adhesive film formulation including at least one film layer containing a prophylactic or therapeutic agent for an animal disease and a highly mucoadhesive, easily water-soluble polymer.

## Description

### Technical Field

The present invention relates to an animal oral cavity-adhesive film formulation.

### Background Art

In non-human animals, such as dogs and cats, the number of oral diseases, such as gingivitis, periodontal disease, and dental caries, has been increasing as in humans, and hence their prevention and treatment have been performed. For example, tooth brushing, the application of ointment or the like, and the oral administration of a supplement, such as a tablet or a gel, have each been performed as means for the prevention and treatment. In addition, for example, toothpaste for suppressing bad breath caused by such oral disease has been on sale. In addition, a composition for preventing and treating periodontal disease in a dog has been reported (Patent Literature 1).

In addition, the number of diseases similar to human lifestyle-related diseases tends to increase year by year in the non-human animals, such as dogs and cats.

### Citation List

### Patent Literature

[PTL 1] JP-A-2009-173558

### Summary of Invention

### Technical Problem

However, a related-art veterinary drug has been administered by an owner as follows: the drug is mixed with food or drink and fed to an animal, or is administered as a tablet or gel. Accordingly, the retention time of the drug in the oral cavity of the animal is short, and hence it has been impossible to sufficiently obtain preventing and treating effects particularly on oral diseases. In addition, even when an oral ointment or the like is administered to the animal, the ointment is easily dissolved by saliva or the like, and a sufficient effect has not been obtained.

Accordingly, the present invention aims to provide a veterinary formulation that can retain a drug in the oral cavity of an animal for a sufficient time period or can quickly release the drug in the oral cavity of the animal.

### Solution to Problem

In view of the foregoing, the present inventors found that an animal oral cavity-adhesive film formulation, which was able to adhere to gums in the oral cavity of an animal for a long time period and quickly release a drug, was obtained by including a veterinary drug in a film formulation that was able to be caused to adhere to the gums in the oral cavity of the animal. Thus, the present inventors completed the present invention.

That is, the present invention provides the following inventions [1] to [8].
[1] An animal oral cavity-adhesive film formulation, comprising at least one film layer containing a prophylactic or therapeutic agent for an animal disease and an easily water-soluble polymer.
[2] The film formulation according to [1], wherein the easily water-soluble polymer is one or two or more kinds selected from pullulan, starch, xanthan gum, sodium alginate, polyvinylpyrrolidone, hydroxypropylcellulose, hydroxypropylmethylcellulose, methylcellulose, sodium carboxymethylcellulose, polyvinyl alcohol, polyacrylic acid, carboxyvinyl polymer, and hydroxyethylcellulose.
[3] The film formulation according to [1] or [2], wherein the film formulation is an animal oral cavity-adhesive film formulation comprising two or more film layers in which an outermost film layer is a film layer containing the prophylactic or therapeutic agent for an animal disease and the easily water-soluble polymer, or a film layer containing the easily water-soluble polymer.
[4] The film formulation according to any one of [1] to [3], wherein the film formulation has a thickness of from 50 µm to 1,000 µm.
[5] An animal oral cavity-adhesive film formulation, comprising at least one film layer containing a prophylactic or therapeutic agent for an animal disease and a highly mucoadhesive, easily water-soluble polymer.
[6] The film formulation according to [5], wherein the film formulation is an animal oral cavity-adhesive film formulation comprising two or more film layers in which an outermost film layer is a film layer containing the prophylactic or therapeutic agent for an animal disease and the highly mucoadhesive, easily water-soluble polymer, or a film layer containing the highly mucoadhesive, easily water-soluble polymer.
[7] The film formulation according to [5] or [6], wherein the highly mucoadhesive, easily water-soluble polymer is one or two or more kinds selected from pullulan, polyvinylpyrrolidone, polyacrylic acid, and carboxyvinyl polymer.
[8] The film formulation according to any one of [5] to [7], wherein the film formulation has a thickness of from 50 µm to 1,000 µm.

### Advantageous Effects of Invention

The animal oral cavity-adhesive film formulation of the present invention quickly adheres to the oral mucosa of a non-human animal, and quickly releases its prophylactic or therapeutic agent for an animal disease. Accordingly, the drug is retained in the oral cavity of the animal for a long time period, and hence a preventing or treating effect particularly on an oral disease of the animal is reliably obtained. In addition, the animal oral cavity-adhesive film formulation of the present invention is extremely thin, and hence can be caused to adhere to the gums of the animal. Accordingly, the animal does not try to remove the film, and hence the drug can be released for a long time period.

### Brief Description of Drawings

FIG. 1 shows the results of a periodontitis score evaluation.
FIG. 2 shows the results of a mouth odor sensory score evaluation.
FIG. 3 shows evaluation results for an odor type A (a putrefactive odor (sulfur compound-based odor: an odor related to the degree of periodontal disease)).
FIG. 4 shows evaluation results for an odor type B (a slightly fishy odor (an odor related to the amount of saliva secretion)).
FIG. 5 shows the results of an evaluation using OraStrip (with the concentration of thymol in a mouth as an index).
FIG. 6 shows the results of an evaluation using ADplit (with the concentration of N-benzoyl-DL-arginyl peptidase in the mouth as an index).

### Description of Embodiments

The terms used herein are each used in a sense typically used in the field unless otherwise specified.

The term "animal" as used herein means a non-human animal, preferably a non-human mammal kept by a human, and examples thereof may include a dog, a cat, a hamster, a rabbit, a degu, a guinea pig, a chinchilla, a pig, a cow, a horse, and a donkey.

The term "oral cavity" as used herein means a space in a mouth, and specific examples thereof may include gums, jaws, palates, cheeks, oral mucosa, and salivary glands in the mouth.

The term "adhesion" as used herein means that an object sticks to another object, and the phrase "a film adheres to a mucosa" indicates that the film sticks for a certain time period.

The term "film" as used herein means a thin membrane, and a film formulation means a formulation in the form of a thin membrane. The thickness of the film is preferably from 50 µm to 1,000 µm, more preferably from 70 µm to 500 µm, still more preferably from 100 µm to 400 µm.

The term "easily water-soluble polymer" as used herein refers to a polymer having the following property: under an environment at 1 atm and 23°C, 1 g of the polymer is weighed, and is then immersed in 10 g of ion-exchanged water; and after the lapse of 24 hours, 0.5 g or more of the immersed polymer dissolves in the water. In the present invention, any easily water-soluble and edible polymer may be suitably used. Specific examples thereof may include one or two or more kinds selected from pullulan, starch, xanthan gum, sodium alginate, polyvinylpyrrolidone, hydroxypropylcellulose, hydroxypropylmethylcellulose, methylcellulose, sodium carboxymethylcellulose, polyvinyl alcohol, polyacrylic acid, carboxyvinyl polymer, and hydroxyethylcellulose.

The term "highly mucoadhesive, easily water-soluble polymer" as used herein refers to a polymer among the above-mentioned easily water-soluble polymers that absorbs moisture from the mucosa to thicken thereby exhibiting high adhesiveness to the mucosa. That is, the polymer is, for example, a polymer exhibiting a high thickening property among the easily water-soluble polymers. Specifically, the polymer is preferably one or two or more kinds selected from pullulan, polyvinylpyrrolidone, polyacrylic acid, and carboxyvinyl polymer.

The term "prophylactic or therapeutic agent for an animal disease" as used herein may refer to any drug that prevents or treats various diseases in the animal, and examples thereof may include various oral care active ingredients, and various disease prophylactic and therapeutic drugs. Herein, examples of the animal disease may include the diseases of the non-human animal, and examples thereof may include oral diseases, digestive system diseases, liver diseases, kidney diseases, central nervous system diseases, circulatory system diseases in a heart, blood vessels, and the like, and infectious diseases. However, the drug is preferably intended for oral diseases. Examples of the oral diseases may include gingivitis, periodontal disease, dental caries, and stomatitis.

Examples of the oral care active ingredients may include: bacterial cells, such as lactic acid bacteria and lactic acid bacteria (dead cells); protein-based active ingredients, such as lactoferrin and lactoperoxidase; plant-extracted extracts, such as bay leaf extract, a complex extract of licorice and balloon flower roots, and licorice extract; and sugar alcohols, such as erythritol, sorbitol, and xylitol. In addition, the examples may include: components for improving an environment in an oral cavity, such as eriodictyol-6-C-glucoside, epigallocatechin gallate, reduced coenzyme Q10, and green tea fluorine; components for suppressing inflammation, such as lysozyme chloride, glycyrrhizinic acid, glycyrrhetinic acid, sodium azulenesulfonate, lithospermum extract, meloxicam, aspirin aluminum, anhydrous caffeine, ascorbic acid, and tocopherol succinate; bactericidal components, such as cetylpyridinium chloride, benzethonium chloride, benzalkonium chloride, chlorhexidine hydrochloride, decalinium, isopropylmethylphenol, clavulanic acid, potassium clavulanate, and interferon α; and antibiotics, such as azithromycin, tetracycline hydrochloride, amoxicillin hydrate, clindamycin hydrochloride, minocycline, spiramycin, metronidazole, and pradofloxacin.

Further, examples other than the oral care active ingredients may include: nervous system drugs, such as aspirin aluminum, anhydrous caffeine, meloxicam, robenacoxib, grapiprant, ketoprofen, zonisamide, mavacoxib, firocoxib, imepitoin, fluoxetine, and carprofen; circulatory and respiratory system drugs, such as alacepril, temocapril hydrochloride, enalapril maleate, dried extract of Mokuboi-to, telmisartan, pimobendan, benazepril hydrochloride, beraprost sodium, torasemide, allopurinol, amlodipine, candesartan, and beraprost sodium; gastrointestinal drugs, such as maropitant citrate monohydrate, berberine tannate, bismuth subnitrate, dried extract of Geranium herb, nutgall powder, belladonna extract powder, ursodeoxycholic acid, Phellodendron bark powder, biodiastase, biotamylase, lipase, aluminum hydroxynaphthoate, Nux vomica extract, cinnamon powder, fennel powder, acrinol, albumin tannate, sporulating lactic acid bacteria, pancreatin, betaine hydrochloride, saccharated pepsin, cellulase, saccharifying bacteria, mosapride citrate hydrate, aluminum silicate, and metoclopramide hydrochloride; liver disease drugs/detoxicants such as Kremezin; metabolic drugs, such as cyclosporine, trilostane, oclacitinib maleate, osaterone acetate, levothyroxine sodium hydrate, and iron dextran; synthetic antibacterial agents, such as enrofloxacin, marbofloxacin, ofloxacin, and orbifloxacin; antibiotics, such as prednisolone, clindamycin, erythromycin, gentamicin sulfate, betamethasone valerate, clotrimazole, amoxicillin hydrate, potassium clavulanate, cefpodoxime proxetil, cephalexin, and tilmicosin; internal parasite control agents, such as ivermectin, piperazine citrate, santonin, bromofenofos, Macleaya cordata, kamala, Actinidia polygama, pyrantel pamoate, melarsomine dihydrochloride, milbemycin oxime, praziquantel, selamectin, eprinomectin, dichlorophen, lufenuron, piperamine adipate, disophenol, emodepside, toltrazuril, flubendazole, febantel, and moxidectin; liver disease drugs/detoxicants, such as aminoethylsulfonic acid, thiamine hydrochloride, pyridoxine hydrochloride, DL-methionine, thiamine nitrate, riboflavin phosphate sodium, spherical activated carbon, choline chloride, nicotinamide, taurine, and cyanocobalamin; vitamin agents, such as vitamin A, vitamin D3, thiamine nitrate, riboflavin, pyridoxine hydrochloride, cyanocobalamin, tocopherol acetate, aminoethylsulfonate, calcium pantothenate, nicotinic acid, and nicotinamide; antineoplastic agents, such as interferon and toceranib phosphate; food materials each having an intestinal regulatory effect, such as lactic acid bacteria (Lactobacillus as Lactobacilli, Lactococcus and Streptococcus as lactic acid cocci), and Bifidobacterium (Bifidobacterium); and food materials each supporting a kidney function, such as calcium carbonate, chitosan, and a phosphorus adsorbent.

One aspect of the present invention is directed to an animal oral cavity-adhesive film formulation including at least one film layer containing a prophylactic or therapeutic agent for an animal disease and an easily water-soluble polymer.

Hereinafter, a film layer containing the prophylactic or therapeutic agent for an animal disease may be simply referred to as "drug layer," and a film layer that is free of the prophylactic or therapeutic agent for an animal disease may be simply referred to as "coating layer."

The film formulation of the present invention may be a single-layer formulation or a multi-layer formulation. That is, the film formulation may be a single-layer formulation in which all active ingredients are added to one layer, or may be a multi-layer formulation in which one layer is formed for each active ingredient and the layers are bonded to each other to form a single sheet. In addition, a plurality of film layers each containing the easily water-soluble polymer (film layers that are each free of the prophylactic or therapeutic agent for an animal disease) (coating layers) and a plurality of drug layers may be combined.

That is, while the film formulation of the present invention has only to include at least one film layer containing the prophylactic or therapeutic agent for an animal disease and the easily water-soluble polymer, the film formulation may be an animal oral cavity-adhesive film formulation including two or more film layers in which the outermost film layer is a film layer containing the prophylactic or therapeutic agent for an animal disease and the easily water-soluble polymer, or a film layer containing the easily water-soluble polymer (film layer that is free of the prophylactic or therapeutic agent for an animal disease). More specifically, examples thereof may include (1) a film formulation including one to three film layers each containing the prophylactic or therapeutic agent for an animal disease and the easily water-soluble polymer, and (2) a film formulation including one to three film layers each containing the prophylactic or therapeutic agent for an animal disease and the easily water-soluble polymer, and including a film layer containing the easily water-soluble polymer (film layer that is free of the prophylactic or therapeutic agent for an animal disease) on one surface, or each of both the surfaces, thereof.

In addition, when a protein-based active ingredient is present in the plurality of active ingredients, the film formulation is preferably formed so as to include the following two kinds of layers: a layer having added thereto the protein-based active ingredient (drug layer I) and a layer containing any other active ingredient (drug layer II). The reason for the foregoing is to prevent the denaturation and deactivation of the active ingredients due to a solvent to be used in the production of the film formulation or an influence of thermal load. The formulation may have the configuration "(drug layer I)/(drug layer II)/(drug layer I)," or may have the configuration "(drug layer II)/(drug layer I)/(drug layer II)." Further, a more complex multi-layer structure may be used. In addition, when some of the active ingredients each have a bitter taste, a layer that masks the bitter taste may be disposed on each of both the surfaces of the film formulation or between the layers thereof.

First, the "film layer containing the prophylactic or therapeutic agent for an animal disease and the easily water-soluble polymer" (drug layer) is described.

The film layer (drug layer) contains the prophylactic or therapeutic agent for an animal disease.

Examples of the animal disease may include such systemic diseases as described above. However, the drug is preferably intended for oral diseases, such as gingivitis, periodontal disease, dental caries, and stomatitis. In recent years, the eating habits of pet animals have changed, and hence the number of those oral diseases similar to those of humans has been increasing. Accordingly, the development of effective prophylactic and therapeutic agents for those oral diseases has been desired. The number of lifestyle-related diseases (e.g., hypertension, diabetes, heart disease, cerebrovascular disease, liver cirrhosis, chronic renal failure, and cancer) in the pet animals has also been increasing, and hence simple means for administration at home or by a veterinarian has been desired in their prevention and treatment.

The oral care active ingredients are preferred as prophylactic or therapeutic agents for those animal diseases, and examples thereof may include: bacterial cells, such as lactic acid bacteria and lactic acid bacteria (dead cells); protein-based active ingredients, such as lactoferrin and lactoperoxidase; plant-extracted extracts, such as bay leaf extract, a complex extract of licorice and balloon flower roots, and licorice extract; and erythritol. In addition, the following components are preferred: components for improving an environment in an oral cavity, such as eriodictyol-6-C-glucoside, epigallocatechin gallate, reduced coenzyme Q10, and green tea fluorine; components for suppressing inflammation, such as lysozyme chloride, glycyrrhizinic acid, glycyrrhetinic acid, sodium azulenesulfonate, lithospermum extract, meloxicam, aspirin aluminum, anhydrous caffeine, ascorbic acid, and tocopherol succinate; bactericidal components, such as cetylpyridinium chloride, benzethonium chloride, benzalkonium chloride, chlorhexidine hydrochloride, decalinium, isopropylmethylphenol, clavulanic acid, potassium clavulanate, and interferon α; and antibiotics, such as azithromycin, tetracycline hydrochloride, amoxicillin hydrate, clindamycin hydrochloride, minocycline, spiramycin, metronidazole, and pradofloxacin.

The characteristics and preferred dosages of the oral care active ingredients are as described below.

Known examples of the lactic acid bacteria may include: Lactobacillus salivarius, Lactobacillus reuteri, Lactobacillus rhamnosus, and Lactobacillus sporogenes of the Lactobacilli, which are generally said to be effective against periodontal bacteria; and Enterococcus faecalis of the lactic acid cocci. However, sporulating lactic acid bacteria are preferred in order to enhance the stability of the final formulation.

300 million to 400 billion or more lactic acid bacteria, which are said to be effective against periodontal disease, are typically present per 1 g of the raw material powder of the lactic acid bacteria.

The intake of the lactic acid bacteria is desirably 0.0001 mg or more and 200 mg or less, preferably 0.1 mg or more and 100 mg or less, more preferably 1 mg or more and 50 mg or less per day for a dog or cat having a body weight of approximately 10 kg, preferably 8 kg or more and less than 15 kg.

In addition, in the case of a dog or cat having a body weight of approximately 5 kg, preferably less than 8 kg, the intake is desirably 0.00005 mg or more and 100 mg or less, preferably 0.05 mg or more and 50 mg or less, more preferably 0.5 mg or more and 25 mg or less per day.

In the case of a dog or cat having a body weight of approximately 20 kg, preferably 15 kg or more, the intake is desirably 0.0002 mg or more and 400 mg or less, preferably 0.2 mg or more and 200 mg or less, more preferably 2 mg or more and 100 mg or less per day.

When live lactic acid bacteria are used, the lactic acid bacteria are gradually deactivated during a production process for the film formulation, and by their storage environment and their storage over time. In contrast, dead cells are easy to handle because the cells are highly stable during the formulation process and at the time of their storage while maintaining their effect. Dead cells of Lactobacillus crispatus have been generally known, and their intake is desirably 0.1 mg or more and 200 mg or less, preferably 1 mg or more and 100 mg or less, more preferably 3 mg or more and 50 mg or less per day for a dog or cat having a body weight of approximately 10 kg, preferably 8 kg or more and less than 15 kg.

In addition, in the case of a dog or cat having a body weight of approximately 5 kg, preferably less than 8 kg, the intake is desirably 0.05 mg or more and 100 mg or less, preferably 0.5 mg or more and 50 mg or less, more preferably 1.5 mg or more and 25 mg or less per day.

In the case of a dog or cat having a body weight of approximately 20 kg, preferably 15 kg or more, the intake is desirably 0.2 mg or more and 400 mg or less, preferably 2 mg or more and 200 mg or less, more preferably 6 mg or more and 100 mg or less per day.

Lactoferrin is present in saliva. Lactoferrin binds to bacteria and is digested or spit out with the saliva. It has been generally known that when the total amount of components for forming the saliva is set to 100, lactoferrin is present at a ratio of from 9 to 24.

In addition, it has been said that the daily amount of saliva in a human reaches from 1 L to 1.5 L, and that in a dog having a body weight of 20 kg reaches 3 L. A proliferation tendency of bad bacteria is observed in approximately half a day in a circadian rhythm, which is a time wave during which the bad bacteria are liable to occur in an oral cavity. When the fact that a difference in component amount between a human and a dog is 10:1 on average is used as a guide, the required amount of lactoferrin can be calculated to be approximately 15 mg for a dog having a body weight of 20 kg.

In other words, the intake of lactoferrin is desirably 1 mg or more and 100 mg or less, preferably 2 mg or more and 50 mg or less, more preferably 3 mg or more and 25 mg or less per day for a dog or cat having a body weight of approximately 10 kg, preferably 8 kg or more and less than 15 kg.

In addition, in the case of a dog or cat having a body weight of approximately 5 kg, preferably less than 8 kg, the intake is desirably 0.5 mg or more and 50 mg or less, preferably 1 mg or more and 25 mg or less, more preferably 2 mg or more and 12.5 mg or less per day.

In the case of a dog or cat having a body weight of approximately 20 kg, preferably 15 kg or more, the intake is desirably 2 mg or more and 200 mg or less, preferably 4 mg or more and 100 mg or less, more preferably 8 mg or more and 50 mg or less per day.

As in lactoferrin, lactoperoxidase is present in saliva and has strong bactericidal activity.

It has been generally known that when the total amount of components for forming the saliva is set to 100, lactoperoxidase is present at a ratio of 2.

It has been said that the daily amount of saliva in a human reaches from 1 L to 1.5 L, and that in a dog having a body weight of 20 kg reaches 3 L. A proliferation tendency of bad bacteria is observed in approximately half a day in a circadian rhythm, which is a time wave during which the bad bacteria are liable to occur in an oral cavity. In addition, when the fact that a difference in component amount between a human and a dog is 10:1 on average is used as a guide, the required amount of lactoperoxidase can be calculated to be from approximately 1 mg to approximately 2 mg for a dog having a body weight of 20 kg.

In other words, the intake of lactoperoxidase is desirably 0.1 mg or more and 20 mg or less, preferably 0.2 mg or more and 10 mg or less, more preferably 0.5 mg or more and 5 mg or less per day for a dog or cat having a body weight of approximately 10 kg, preferably 8 kg or more and less than 15 kg.

In addition, in the case of a dog or cat having a body weight of approximately 5 kg, preferably less than 8 kg, the intake is desirably 0.05 mg or more and 10 mg or less, preferably 0.1 mg or more and 5 mg or less, more preferably 0.25 mg or more and 2.5 mg or less per day.

In the case of a dog or cat having a body weight of approximately 20 kg, preferably 15 kg or more, the intake is desirably 0.2 mg or more and 40 mg or less, preferably 0.4 mg or more and 20 mg or less, more preferably 1 mg or more and 10 mg or less per day.

Bay leaf extract may be used as a dried product, which is obtained by extraction with water or warm water, an alcohol-based solvent, acetone, or any other organic solvent, followed by the concentration or drying of the filtrate, or as a crude product or a purified product.

The intake of the bay leaf extract is desirably 0.5 mg or more and 50 mg or less, preferably 1 mg or more and 30 mg or less, more preferably 2 mg or more and 20 mg or less per day for a dog or cat having a body weight of approximately 10 kg, preferably from 8 kg to 15 kg.

In the case of a dog or cat having a body weight of approximately 5 kg, preferably less than 8 kg, the intake is desirably 0.25 mg or more and 25 mg or less, preferably 0.5 mg or more and 15 mg or less, more preferably 1 mg or more and 10 mg or less per day.

In the case of a dog or cat having a body weight of approximately 20 kg, preferably 15 kg or more, the intake is desirably 1 mg or more and 400 mg or less, preferably 2 mg or more and 200 mg or less, more preferably 4 mg or more and 100 mg or less per day.

A complex extract of licorice and balloon flower roots may be used as a dried product, which is obtained by the extraction of a licorice root and a balloon flower root with water or warm water, an alcohol-based solvent, acetone, or any other organic solvent, followed by the concentration or drying of the filtrate, or as a crude product or a purified product.

The intake of the extract is desirably 1 mg or more and 200 mg or less, preferably 2 mg or more and 100 mg or less, more preferably 5 mg or more and 50 mg or less per day for a dog or cat having a body weight of approximately 10 kg, preferably from 8 kg to 15 kg.

In the case of a dog or cat having a body weight of approximately 5 kg, preferably less than 8 kg, the intake is desirably 0.5 mg or more and 100 mg or less, preferably 1 mg or more and 50 mg or less, more preferably 2 mg or more and 25 mg or less per day.

In the case of a dog or cat having a body weight of approximately 20 kg, preferably 15 kg or more, the intake is desirably 2 mg or more and 400 mg or less, preferably 4 mg or more and 200 mg or less, more preferably 10 mg or more and 100 mg or less per day.

Erythritol is a saccharide known to have a suppressing effect on plaque accumulation and a promoting effect on the bactericidal action of saliva in a human. A similar effect is expected in a dog.

The intake of erythritol is desirably 0.01 mg or more and 800 mg or less, preferably 0.1 mg or more and 400 mg or less, more preferably 1 mg or more and 200 mg or less per day for a dog or cat having a body weight of approximately 10 kg, preferably from 8 kg to 15 kg.

In the case of a dog or cat having a body weight of approximately 5 kg, preferably less than 8 kg, the intake is desirably 0.005 mg or more and 400 mg or less, preferably 0.05 mg or more and 200 mg or less, more preferably 0.5 mg or more and 100 mg or less per day.

In the case of a dog or cat having a body weight of approximately 20 kg, preferably 15 kg or more, the intake is desirably 0.02 mg or more and 1,600 mg or less, preferably 0.2 mg or more and 800 mg or less, more preferably 2 mg or more and 400 mg or less per day.

In addition, xylitol, which is also a sugar alcohol as in erythritol, may also be used because xylitol is known to have a suppressing effect on the proliferation of periodontal bacteria and a suppressing effect on plaque accumulation in a human.

The easily water-soluble and edible polymer may be suitably used as the easily water-soluble polymer. Specific examples thereof may include one or two or more kinds selected from pullulan, starch, xanthan gum, sodium alginate, polyvinylpyrrolidone, hydroxypropylcellulose, hydroxypropylmethylcellulose, methylcellulose, sodium carboxymethylcellulose, polyvinyl alcohol, polyacrylic acid, carboxyvinyl polymer, and hydroxyethylcellulose. Of those, from the viewpoint of the characteristic of being quickly thickened and dissolved by a mucosa or saliva, one or two or more kinds selected from pullulan, starch, polyvinylpyrrolidone, hydroxypropylcellulose, hydroxypropylmethylcellulose, sodium carboxymethylcellulose, polyvinyl alcohol, polyacrylic acid, carboxyvinyl polymer, and hydroxyethylcellulose are more preferred, one or two or more kinds selected from pullulan, polyvinylpyrrolidone, hydroxypropylcellulose, hydroxypropylmethylcellulose, sodium carboxymethylcellulose, polyvinyl alcohol, polyacrylic acid, carboxyvinyl polymer, and hydroxyethylcellulose are still more preferred, and one or two or more kinds selected from hydroxypropylcellulose and hydroxypropylmethylcellulose are still further more preferred.

Hydroxypropylcellulose is suitable because the combination of its grades having different degrees of polymerization can change the drug release rate of the film formulation. Specific examples thereof may include HPC-SSL, HPC-SL, HPC-L, HPC-M, and HPC-H from Nippon Soda Co., Ltd.

Hydroxypropylmethylcellulose is suitable because the dissolution rate and drug release rate of the film formulation can be changed by the degrees to which hydroxypropylmethylcellulose is substituted with a methoxy group and a hydroxypropoxy group. Specific examples thereof may include TC-5E, TC-5M, TC-5R, and TC-5S from Shin-Etsu Chemical Co., Ltd.

Hydroxypropylmethylcellulose (HPMC) has hygroscopicity lower than that of hydroxypropylcellulose (HPC). Accordingly, when the film formulation is a multi-layer type, the main water-soluble polymer of its outer layer is preferably hydroxypropylmethylcellulose.

The easily water-soluble polymer is incorporated into the drug-containing film layer of the film formulation at preferably from 20 mass% to 70 mass%, more preferably from 25 mass% to 60 mass% from the viewpoints of, for example, the solubility of the drug-containing film layer and the retentivity of the drug.

In addition to the above-mentioned components, a plasticizer, an antioxidant, a sweetener, a coloring agent, or the like may be incorporated into the drug layer.

The term "plasticizer" means a substance that is highly compatible with the easily water-soluble polymer and imparts flexibility to the drug-containing film layer. Examples of the plasticizer may include polyethylene glycol, glycerin, propylene glycol, triacetin, triethyl citrate, and castor oil. Polyethylene glycol is suitable because polyethylene glycol comes in grades having different average molecular weights, and hence facilitates the adjustment of the flexibility of the drug-containing film layer. The content of the plasticizer is preferably within 20 mass% in the drug-containing film layer in terms of ease of handling.

Examples of the antioxidant may include L-ascorbyl stearate, ascorbic acid, sodium sulfite, sodium edetate, erythorbic acid, tocopherol acetate, dibutylhydroxytoluene, vitamin E, tocopherol, and butylhydroxyanisole.

For example, aspartame, stevia, sucralose, glycyrrhizinic acid and a salt thereof, thaumatin, acesulfame potassium, and saccharin and a salt thereof may each be used as the sweetener.

A coloring agent may be added to the film formulation in order to enhance the distinctiveness of the formulation, to make it easier to recognize the state of the formulation during its application to gums, or to make the formulation during the application to the gums inconspicuous. The coloring agent is not particularly limited as long as the coloring agent may be typically used in a pharmaceutical. Examples thereof may include tar-based dyes and aluminum lakes thereof specified by Ministerial Ordinance No. 30 of the Ministry of Health and Welfare. In addition, the examples may include ferric oxide and yellow ferric oxide.

Further, an opacifying agent, such as titanium oxide, talc, zinc oxide, or magnesium oxide, may be added to opacify the film formulation.

In addition, the coloring agent and the opacifying agent may be added to the same layer. Further, distinctiveness between the front and back surfaces of the film formulation can be improved and the appearance thereof can be enhanced by combining a colored layer and an opacified layer, and optimizing the layer configuration of the formulation.

Next, the film layer containing the easily water-soluble polymer (film layer that is free of the prophylactic or therapeutic agent for an animal disease) (coating layer) is described.

The coating layer is basically a film layer containing the easily water-soluble polymer, and the easily water-soluble polymer to be used is preferably the same as that blended into the drug layer.

The easily water-soluble polymer is incorporated into the coating layer at preferably from 60 mass% to 100 mass%, more preferably from 80 mass% to 100 mass% from the viewpoint of the solubility of the coating layer.

In addition to the easily water-soluble polymer, a compound that promotes the solubility such as a surfactant component may be incorporated into the coating layer.

The compound that promotes the solubility such as a sugar alcohol is desirably added to accelerate the dissolution of the coating layer so that the coating layer may more quickly exhibit its adhesiveness when the formulation is brought into contact with the mucosal surface of the gums. Specific examples thereof may include sorbitol, maltitol, xylitol, and mannitol. Of those, sorbitol is desired because sorbitol not only promotes the solubility but also imparts favorable flexibility to the formulation.

In order for the coating layer to dissolve quickly and exhibit the adhesiveness, the surfactant component is desirably incorporated so that a component (e.g., saliva) on the mucosal surface and the surface of the coating layer may be quickly mixed. Examples of the surfactant component may include a sucrose fatty acid ester, sodium lauryl sulfate, a glycerin fatty acid ester, a polyoxyethylene glycerin fatty acid ester, a sorbitan fatty acid ester, a polyoxyethylene sorbitan fatty acid ester, and polyoxyethylene hydrogenated castor oil. A sucrose fatty acid ester is more preferred in terms of an influence on the taste of the formulation and the mucosal irritancy thereof.

The size and shape of the film formulation of the present invention are not particularly limited as long as the formulation is easy to handle at the time of its administration and an animal to be administered does not resist. Examples of the shape may include a quadrangle, a quadrangle with rounded corners, a circular shape, and an elliptical shape. With regard to a ratio between the short side (minor axis) and long side (major axis) of the formulation, the long side (major axis) is preferably from 1 to 5, more preferably from 1 to 4, still more preferably from 1 to 3 when the short side (minor axis) is defined as 1.

In addition, an incision (notch) or a perforation may be made to enable the adjustment of the size of the film formulation through its cutting so that its dosage can be adjusted. Possible shapes of the incision may include a "V-shape" and an "I-shape".

In addition, a sheet-like material before its cutting into individual film formulations may be cut into an appropriate size and used by a user with reference to, for example, the body weight of an animal serving as a guide. A grid pattern (lattice pattern) may be printed on the surface of the sheet with an edible ink. For example, the formulation is cut into a size of 1 square per 1 kg of the body weight of the animal and administered.

The thickness of the formulation is preferably from 50 µm to 1,000 µm, more preferably from 70 µm to 800 µm, still more preferably from 100 µm to 500 µm. When the thickness falls within the ranges, the ease of handling of the formulation at the time of its administration and the solubility of the formulation after the administration are satisfactory.

Another aspect of the present invention is directed to an animal oral cavity-adhesive film formulation including at least one film layer containing a prophylactic or therapeutic agent for an animal disease and a highly mucoadhesive, easily water-soluble polymer.

The film formulation of the present invention may be a single-layer formulation or a multi-layer formulation. That is, the film formulation may be a single-layer formulation in which all active ingredients are added to one layer, or may be a multi-layer formulation in which one layer is formed for each active ingredient and the layers are bonded to each other to form a single sheet. In addition, a plurality of film layers each containing the easily water-soluble polymer (film layers that are each free of the prophylactic or therapeutic agent for an animal disease) (coating layers) and a plurality of drug layers may be combined.

That is, while the film formulation of the present invention has only to include at least one film layer containing the prophylactic or therapeutic agent for an animal disease and the highly mucoadhesive, easily water-soluble polymer, the film formulation may be an animal oral cavity-adhesive film formulation including two or more film layers in which the outermost film layer is a film layer containing the prophylactic or therapeutic agent for an animal disease and the highly mucoadhesive, easily water-soluble polymer, or a film layer containing the highly mucoadhesive, easily water-soluble polymer (film layer that is free of the prophylactic or therapeutic agent for an animal disease). More specifically, examples thereof may include (1) a film formulation including one to three film layers each containing the prophylactic or therapeutic agent for an animal disease and the highly mucoadhesive, easily water-soluble polymer, and (2) a film formulation including one to three film layers each containing the prophylactic or therapeutic agent for an animal disease and the highly mucoadhesive, easily water-soluble polymer, and including a film layer containing the highly mucoadhesive, easily water-soluble polymer (film layer that is free of the prophylactic or therapeutic agent for an animal disease) on one surface, or each of both the surfaces, thereof.

In addition, when a protein-based active ingredient is present in the plurality of active ingredients, the film formulation is preferably configurated so as to include the following two kinds of layers: a layer to which the protein-based active ingredient has been added (drug layer I) and a layer containing any other active ingredient (drug layer II). The reason for the foregoing is to prevent the denaturation and deactivation of the active ingredients due to a solvent to be used in the production of the film formulation or an influence of thermal load. The formulation may have the configuration "(drug layer I)/(drug layer II)/(drug layer I)," or may have the configuration "(drug layer II)/(drug layer I)/(drug layer II)." Further, a more complex multi-layer structure may be used. In addition, when some of the active ingredients each have a bitter taste, a layer that masks the bitter taste may be disposed on each of both the surfaces of the film formulation or between the layers thereof.

First, the "film layer containing the prophylactic or therapeutic agent for an animal disease and the highly mucoadhesive, easily water-soluble polymer" (drug layer) is described.

The film layer contains the prophylactic or therapeutic agent for an animal disease.

Examples of the animal disease may include such systemic diseases as described above. However, the drug is preferably intended for oral diseases, such as gingivitis, periodontal disease, dental caries, and stomatitis. In recent years, the eating habits of pet animals have changed, and hence the number of those oral diseases similar to those of humans has been increasing. Accordingly, the development of effective prophylactic and therapeutic agents for those oral diseases has been desired. The number of lifestyle-related diseases (e.g., hypertension, diabetes, heart disease, cerebrovascular disease, liver cirrhosis, chronic renal failure, and cancer) in the pet animals has also been increasing, and hence simple means for administration at home or by a veterinarian has been desired in their prevention and treatment.

The oral care active ingredients are preferred as prophylactic or therapeutic agents for those animal diseases, and examples thereof may include: bacterial cells, such as lactic acid bacteria and lactic acid bacteria (dead cells); protein-based active ingredients, such as lactoferrin and lactoperoxidase; plant-extracted extracts, such as bay leaf extract, a complex extract of licorice and balloon flower roots, and licorice extract; and erythritol. In addition, the following components are preferred: components for improving an environment in an oral cavity, such as eriodictyol-6-C-glucoside, epigallocatechin gallate, reduced coenzyme Q10, and green tea fluorine; components for suppressing inflammation, such as lysozyme chloride, glycyrrhizinic acid, glycyrrhetinic acid, sodium azulenesulfonate, lithospermum extract, meloxicam, aspirin aluminum, anhydrous caffeine, ascorbic acid, and tocopherol succinate; bactericidal components, such as cetylpyridinium chloride, benzethonium chloride, benzalkonium chloride, chlorhexidine hydrochloride, decalinium, isopropylmethylphenol, clavulanic acid, potassium clavulanate, and interferon α; and antibiotics, such as azithromycin, tetracycline hydrochloride, amoxicillin hydrate, clindamycin hydrochloride, minocycline, spiramycin, metronidazole, and pradofloxacin.

The characteristics and preferred dosages of the oral care active ingredients are as described below.

Examples of lactic acid bacteria may include: Lactobacillus salivarius, Lactobacillus reuteri, Lactobacillus rhamnosus, and Lactobacillus sporogenes of the Lactobacilli, which are generally known to be effective against periodontal bacteria; and Enterococcus faecalis of the lactic acid cocci. Of those, sporulating lactic acid bacteria are preferred in order to enhance the stability of the final formulation.

300 million to 400 billion or more lactic acid bacteria, which are said to be effective against periodontal disease, are typically present per 1 g of the raw material powder of the lactic acid bacteria.

The intake of the lactic acid bacteria is desirably 0.0001 mg or more and 200 mg or less, preferably 0.1 mg or more and 100 mg or less, more preferably 1 mg or more and 50 mg or less per day for a dog or cat having a body weight of approximately 10 kg, preferably 8 kg or more and less than 15 kg.

In addition, in the case of a dog or cat having a body weight of approximately 5 kg, preferably less than 8 kg, the intake is desirably 0.00005 mg or more and 100 mg or less, preferably 0.05 mg or more and 50 mg or less, more preferably 0.5 mg or more and 25 mg or less per day.

In the case of a dog or cat having a body weight of approximately 20 kg, preferably 15 kg or more, the intake is desirably 0.0002 mg or more and 400 mg or less, preferably 0.2 mg or more and 200 mg or less, more preferably 2 mg or more and 100 mg or less per day.

When live lactic acid bacteria are used, the lactic acid bacteria are gradually deactivated during a production process for the film formation, and by their storage environment and their storage over time. In contrast, dead cells are easy to handle because the cells are highly stable during the formulation process and at the time of their storage while maintaining their effect. Dead cells of Lactobacillus crispatus have been generally known, and their intake is desirably 0.1 mg or more and 200 mg or less, preferably 1 mg or more and 100 mg or less, more preferably 3 mg or more and 50 mg or less per day for a dog or cat having a body weight of approximately 10 kg, preferably 8 kg or more and less than 15 kg.

In addition, in the case of a dog or cat having a body weight of approximately 5 kg, preferably less than 8 kg, the intake is desirably 0.05 mg or more and 100 mg or less, preferably 0.5 mg or more and 50 mg or less, more preferably 1.5 mg or more and 25 mg or less per day.

In the case of a dog or cat having a body weight of approximately 20 kg, preferably 15 kg or more, the intake is desirably 0.2 mg or more and 400 mg or less, preferably 2 mg or more and 200 mg or less, more preferably 6 mg or more and 100 mg or less per day.

Lactoferrin is present in saliva. Lactoferrin binds to bacteria and is digested or spit out with the saliva. It has been generally known that when the total amount of components for forming the saliva is set to 100, lactoferrin is present at a ratio of from 9 to 24.

In addition, it has been said that the daily amount of saliva in a human reaches from 1 L to 1.5 L, and that in a dog having a body weight of 20 kg reaches 3 L. A proliferation tendency of bad bacteria is observed in approximately half a day in a circadian rhythm, which is a time wave during which the bad bacteria are liable to occur in an oral cavity. When the fact that a difference in component amount between a human and a dog is 10:1 on average is used as a guide, the required amount of lactoferrin can be calculated to be approximately 15 mg for a dog having a body weight of 20 kg.

In other words, the intake of lactoferrin is desirably 1 mg or more and 100 mg or less, preferably 2 mg or more and 50 mg or less, more preferably 3 mg or more and 25 mg or less per day for a dog or cat having a body weight of approximately 10 kg, preferably 8 kg or more and less than 15 kg.

In addition, in the case of a dog or cat having a body weight of approximately 5 kg, preferably less than 8 kg, the intake is desirably 0.5 mg or more and 50 mg or less, preferably 1 mg or more and 25 mg or less, more preferably 2 mg or more and 12.5 mg or less per day.

In the case of a dog or cat having a body weight of approximately 20 kg, preferably 15 kg or more, the intake is desirably 2 mg or more and 200 mg or less, preferably 4 mg or more and 100 mg or less, more preferably 8 mg or more and 50 mg or less per day.

As in lactoferrin, lactoperoxidase is present in saliva and has strong bactericidal activity.

It has been generally known that when the total amount of components for forming the saliva is set to 100, lactoperoxidase is present at a ratio of 2.

It has been said that the daily amount of saliva in a human reaches from 1 L to 1.5 L, and that in a dog having a body weight of 20 kg reaches 3 L. A proliferation tendency of bad bacteria is observed in approximately half a day in a circadian rhythm, which is a time wave during which the bad bacteria are liable to occur in an oral cavity. In addition, when the fact that a difference in component amount between a human and a dog is 10:1 on average is used as a guide, the required amount of lactoperoxidase can be calculated to be from approximately 1 mg to approximately 2 mg for a dog having a body weight of 20 kg.

In other words, the intake of lactoperoxidase is desirably 0.1 mg or more and 20 mg or less, preferably 0.2 mg or more and 10 mg or less, more preferably 0.5 mg or more and 5 mg or less per day for a dog or cat having a body weight of approximately 10 kg, preferably 8 kg or more and less than 15 kg.

In addition, in the case of a dog or cat having a body weight of approximately 5 kg, preferably less than 8 kg, the intake is desirably 0.05 mg or more and 10 mg or less, preferably 0.1 mg or more and 5 mg or less, more preferably 0.25 mg or more and 2.5 mg or less per day.

In the case of a dog or cat having a body weight of approximately 20 kg, preferably 15 kg or more, the intake is desirably 0.2 mg or more and 40 mg or less, preferably 0.4 mg or more and 20 mg or less, more preferably 1 mg or more and 10 mg or less per day.

Bay leaf extract may be used as a dried product, which is obtained by extraction with water or warm water, an alcohol-based solvent, acetone, or any other organic solvent, followed by the concentration or drying of the filtrate, or as a crude product or a purified product.

The intake of the bay leaf extract is desirably 0.5 mg or more and 100 mg or less, preferably 1 mg or more and 50 mg or less, more preferably 2 mg or more and 25 mg or less per day for a dog or cat having a body weight of approximately 10 kg, preferably from 8 kg to 15 kg.

In the case of a dog or cat having a body weight of approximately 5 kg, preferably less than 8 kg, the intake is desirably 0.25 mg or more and 50 mg or less, preferably 0.5 mg or more and 25 mg or less, more preferably 1 mg or more and 12.5 mg or less per day.

In the case of a dog or cat having a body weight of approximately 20 kg, preferably 15 kg or more, the intake is desirably 1 mg or more and 200 mg or less, preferably 2 mg or more and 100 mg or less, more preferably 4 mg or more and 50 mg or less per day.

A complex extract of licorice and balloon flower roots may be used as a dried product, which is obtained by the extraction of a licorice root and a balloon flower root with water or warm water, an alcohol-based solvent, acetone, or any other organic solvent, followed by the concentration or drying of the filtrate, or as a crude product or a purified product.

The intake of the extract is desirably 1 mg or more and 200 mg or less, preferably 2 mg or more and 100 mg or less, more preferably 5 mg or more and 50 mg or less per day for a dog or cat having a body weight of approximately 10 kg, preferably from 8 kg to 15 kg.

In the case of a dog or cat having a body weight of approximately 5 kg, preferably less than 8 kg, the intake is desirably 0.5 mg or more and 100 mg or less, preferably 1 mg or more and 50 mg or less, more preferably 2 mg or more and 25 mg or less per day.

In the case of a dog or cat having a body weight of approximately 20 kg, preferably 15 kg or more, the intake is desirably 2 mg or more and 400 mg or less, preferably 4 mg or more and 200 mg or less, more preferably 10 mg or more and 100 mg or less per day.

Erythritol is a saccharide known to have a suppressing effect on plaque accumulation and a promoting effect on the bactericidal action of saliva in a human. A similar effect is expected in a dog.

The intake of erythritol is desirably 0.01 mg or more and 800 mg or less, preferably 0.1 mg or more and 400 mg or less, more preferably 1 mg or more and 200 mg or less per day for a dog or cat having a body weight of approximately 10 kg, preferably from 8 kg to 15 kg.

In the case of a dog or cat having a body weight of approximately 5 kg, preferably less than 8 kg, the intake is desirably 0.005 mg or more and 400 mg or less, preferably 0.05 mg or more and 200 mg or less, more preferably 0.5 mg or more and 100 mg or less per day.

In the case of a dog or cat having a body weight of approximately 20 kg, preferably 15 kg or more, the intake is desirably 0.02 mg or more and 1,600 mg or less, preferably 0.2 mg or more and 800 mg or less, more preferably 2 mg or more and 400 mg or less per day.

In addition, xylitol, which is also a sugar alcohol as in erythritol, may also be used because xylitol is known to have a suppressing effect on the proliferation of periodontal bacteria and a suppressing effect on plaque accumulation in a human.

A polymer, which absorbs the moisture of a mucosa to thicken, to thereby exhibit high adhesiveness to the mucosa, out of the easily water-soluble polymers is preferred as the highly mucoadhesive, easily water-soluble polymer.

Specific examples thereof may include one or two or more kinds selected from pullulan, starch, xanthan gum, sodium alginate, polyvinylpyrrolidone, hydroxypropylcellulose, hydroxypropylmethylcellulose, methylcellulose, sodium carboxymethylcellulose, polyvinyl alcohol, polyacrylic acid, carboxyvinyl polymer, and hydroxyethylcellulose. Of those, from the viewpoint of the characteristic of being quickly thickened and dissolved by a mucosa or saliva, one or two or more kinds selected from polyvinylpyrrolidone, hydroxypropylcellulose, hydroxypropylmethylcellulose, sodium carboxymethylcellulose, polyacrylic acid, and carboxyvinyl polymer are more preferred.

Hydroxypropylcellulose is suitable because the combination of its grades having different degrees of polymerization can change the drug release rate of the film formulation. Specific examples thereof may include HPC-SSL, HPC-SL, HPC-L, HPC-M, and HPC-H from Nippon Soda Co., Ltd.

Hydroxypropylmethylcellulose is suitable because the dissolution rate and drug release rate of the film formulation can be changed by the degrees to which hydroxypropylmethylcellulose is substituted with a methoxy group and a hydroxypropoxy group. Specific examples thereof may include TC-5E, TC-5M, TC-5R, and TC-5S from Shin-Etsu Chemical Co., Ltd.

Hydroxypropylmethylcellulose (HPMC) has hygroscopicity lower than that of hydroxypropylcellulose (HPC). Accordingly, when the film formulation is a multi-layer type, the main water-soluble polymer of its outer layer is preferably hydroxypropylmethylcellulose.

Polyvinylpyrrolidone (PVP) is desirably used in order to obtain sustained mucoadhesiveness. Polyvinylpyrrolidone has the following effect: polyvinylpyrrolidone improves a binding property between the components of the film formulation to make the formulation less likely to break. Specific examples thereof may include Kollidon K-30 and Kollidon K-90 from BASF Corporation.

A combined system of hydroxypropylcellulose (HPC) and polyvinylpyrrolidone (PVP) is preferred from the viewpoint of adjusting the adhesiveness of the film formulation to gums and the release rate of its drug. The adhesive strength and release property of the formulation can be adjusted by changing the grade of each of HPC and PVP, and adjusting a mixing ratio therebetween. PVP is added in an amount of preferably from 15 parts by mass to 80 parts by mass, more preferably from 25 parts by mass to 50 parts by mass, more preferably from 30 parts by mass to 40 parts by mass with respect to 100 parts by mass of HPC. When the amount of PVP is small, sufficient adhesiveness may not be obtained. When the amount is large, the formulation may soften over time to cause a problem in its ease of handling at the time of application.

The highly mucoadhesive, easily water-soluble polymer is incorporated into the drug-containing film layer of the film formulation at preferably from 10 mass% to 90 mass%, more preferably from 30 mass% to 90 mass%, still more preferably from 45 mass% to 80 mass% from the viewpoints of, for example, the solubility of the drug-containing film layer and the retentivity of the drug.

In addition to the above-mentioned components, a plasticizer, an antioxidant, a sweetener, a coloring agent, or the like may be incorporated into the drug-containing film layer.

The term "plasticizer" means a substance that is highly compatible with the highly mucoadhesive, easily water-soluble polymer and imparts flexibility to the drug-containing film layer. Examples of the plasticizer may include polyethylene glycol, glycerin, propylene glycol, triacetin, triethyl citrate, and castor oil. Polyethylene glycol is suitable because polyethylene glycol comes in grades having different average molecular weights, and hence facilitates the adjustment of the flexibility of the drug-containing film layer. The content of the plasticizer is preferably within 20 mass% in the drug-containing film layer in terms of ease of handling.

Examples of the antioxidant may include L-ascorbyl stearate, ascorbic acid, sodium sulfite, sodium edetate, erythorbic acid, tocopherol acetate, dibutylhydroxytoluene, vitamin E, tocopherol, and butylhydroxyanisole.

For example, aspartame, stevia, sucralose, glycyrrhizinic acid and a salt thereof, thaumatin, acesulfame potassium, and saccharin and a salt thereof may each be used as the sweetener.

A coloring agent may be added to the film formulation in order to enhance the distinctiveness of the formulation, to make it easier to recognize the state of the formulation during its application to gums, or to make the formulation during the application to the gums inconspicuous. The coloring agent is not particularly limited as long as the coloring agent may be typically used in a pharmaceutical. Examples thereof may include tar-based dyes and aluminum lakes thereof specified by Ministerial Ordinance No. 30 of the Ministry of Health and Welfare. In addition, the examples may include ferric oxide and yellow ferric oxide.

Further, an opacifying agent, such as titanium oxide, talc, zinc oxide, or magnesium oxide, may be added to opacify the film formulation.

In addition, the coloring agent and the opacifying agent may be added to the same layer. Further, distinctiveness between the front and back surfaces of the film formulation can be improved and the appearance thereof can be enhanced by combining a colored layer and an opacified layer, and optimizing the layer configuration of the formulation.

Next, the film layer containing the highly mucoadhesive, easily water-soluble polymer (film layer that is free of the prophylactic or therapeutic agent for an animal disease) (coating layer) is described.

The coating layer is basically a film layer containing the highly mucoadhesive, easily water-soluble polymer, and the highly mucoadhesive, easily water-soluble polymer to be used is preferably the same as that blended into the drug layer. Of those, polyvinylpyrrolidone, polyacrylic acid, carboxyvinyl polymer, or pullulan is preferred in terms of adhesiveness to the mucosal surface of gums. Of those, pullulan is preferred because pullulan quickly exhibits its adhesiveness when brought into contact with the mucosal surface of the gums.

The highly mucoadhesive, easily water-soluble polymer is incorporated into the coating layer at preferably from 60 mass% to 100 mass%, more preferably from 80 mass% to 100 mass% from the viewpoints of the mucoadhesiveness and solubility of the coating layer.

In addition to the highly mucoadhesive, easily water-soluble polymer, a compound that promotes the solubility such as a surfactant component may be incorporated into the coating layer.

The compound that promotes the solubility such as a sugar alcohol is desirably added to accelerate the dissolution of the coating layer so that the coating layer may more quickly exhibit its adhesiveness when the formulation is brought into contact with the mucosal surface of the gums. Specific examples thereof may include sorbitol, maltitol, xylitol, and mannitol. Of those, sorbitol is preferred because sorbitol not only promotes the solubility but also imparts satisfactory flexibility to the formulation.

In order for the coating layer to dissolve quickly and exhibit the adhesiveness, a surfactant component is desirably incorporated so that a component (e.g., saliva) on the mucosal surface and the surface of the coating layer may be quickly mixed. Examples of the surfactant component may include a sucrose fatty acid ester, sodium lauryl sulfate, a glycerin fatty acid ester, a polyoxyethylene glycerin fatty acid ester, a sorbitan fatty acid ester, a polyoxyethylene sorbitan fatty acid ester, and polyoxyethylene hydrogenated castor oil. A sucrose fatty acid ester is more preferred in terms of an influence on the taste of the formulation and the mucosal irritancy thereof.

The size and shape of the film formulation of the present invention are not particularly limited as long as the formulation is easy to handle at the time of its administration and an animal to be administered does not resist. Examples of the shape may include a quadrangle, a quadrangle with rounded corners, a circular shape, and an elliptical shape. With regard to a ratio between the short side (minor axis) and long side (major axis) of the formulation, the long side (major axis) is preferably from 1 to 5, more preferably from 1 to 4, still more preferably from 1 to 3 when the short side (minor axis) is defined as 1.

In addition, an incision (notch) or a perforation may be made to enable the adjustment of the size of the film formulation through its cutting so that its dosage can be adjusted. Possible shapes of the incision may include a "V-shape" and an "I-shape".

In addition, a sheet-like material before its cutting into individual film formulations may be cut into an appropriate size and used by a user with reference to, for example, the body weight of an animal serving as a guide. A grid pattern (lattice pattern) may be printed on the surface of the sheet with an edible ink. For example, the formulation is cut into a size of 1 square per 1 kg of the body weight of the animal and administered.

The thickness of the formulation is preferably from 50 µm to 1,000 µm, more preferably from 70 µm to 800 µm, still more preferably from 100 µm to 500 µm. When the thickness falls within the ranges, the ease of handling of the formulation at the time of its administration and the solubility of the formulation after the administration are satisfactory.

Next, a method of producing the film formulation according to each of both the above-mentioned embodiments of the present invention is described.

The film formulation may be produced by a known method. For example, a film formulation having the three-layer structure "(layer A)/(layer B)/(layer A)" may be produced by each of the following methods.

Method 1: (1) The components of each of the layer A and the layer B are dissolved in a predetermined solvent to provide a layer-A coating liquid or a layer-B coating liquid (coating liquid formulation step). (2) The layer-A coating liquid or the layer-B coating liquid is uniformly applied onto a plastic film and then dried to form the layer A or the layer B (application and drying step). (3) The layer B is bonded onto the layer A obtained in the application and drying step, and the plastic film on the layer B side is peeled off, followed by the bonding of the layer A onto the layer B (lamination step).

Method 2: (1) The layer-A coating liquid is uniformly applied onto a plastic film and then dried to form the layer A. (2) The layer-B coating liquid is uniformly applied onto the layer A and then dried to form the layer B (stacked application and drying step). (3) The materials obtained by the stacked application and drying are bonded to each other so that the layers B may be brought into contact with each other.

Method 3: (1) The layer-A coating liquid is uniformly applied onto a plastic film and then dried to form the layer A. (2) The layer-B coating liquid is uniformly applied onto the layer A and then dried to form the layer B. (3) The layer-A coating liquid is uniformly applied onto the layer B and then dried to form the layer A.

The method 1 is a method of obtaining a predetermined formulation configuration by bonding the respective layers separately formed by the application and drying to each other. The method 3 is a method of sequentially performing the stacked application and drying of the coating liquids for the respective layers so that a predetermined formulation configuration may be obtained. The method 2 is a method obtained by combining the method 1 and the method 3. The methods 1 to 3 may be combined in consideration of, for example, the layer configuration and thickness of a target film formulation, the drying properties of the coating liquids, and the amount of thermal load to the active ingredients of the formulation.

When the layers are bonded (laminated) in a production process for the film formulation, hydroxypropylcellulose (HPC) is preferably incorporated into the water-soluble polymer of each of both, or one, of the layers to be bonded to each other. In each of the above-mentioned methods, when layers obtained from the same coating liquid are bonded to each other, the layers are counted as one layer. In addition, layers obtained by the stacked application and drying of the same coating liquid are also counted as one layer.

When a plurality of layers having the same composition are disposed, for example, in the case of a formulation having the configuration "(layer A)/(layer B)/(layer A)," the thicknesses of the layers A may be identical to or different from each other.

Examples of the coating film (plastic film) may include films made of polyethylene terephthalate (PET), polypropylene (PP), and polyethylene (PE). The surface of the plastic film is preferably subjected to silicone processing or discharge corona processing as required. The performance of such processing enables uniform application of each of the coating liquids and successful removal of the plastic film from the surface of each layer formed after the drying of the coating liquid. A plastic film made of polyethylene terephthalate (PET) is preferred because the occurrence of strain in the application and drying step is suppressed.

The solvent for preparing each of the coating liquids is not particularly limited as long as a coating liquid in which the components of each layer are uniformly dissolved or dispersed can be obtained, and the coating liquid can be uniformly applied and dried. Water, ethanol, and a mixed liquid of water and ethanol are preferred in terms of ease of handling and toxicity.

When any one of the active ingredients is a protein-based active ingredient, such as lactoferrin or lactoperoxidase, water to which ethanol is not added is preferably used in terms of the solubility of the protein-based active ingredient and the avoidance of the denaturation thereof by ethanol. When ethanol needs to be added from the viewpoint of, for example, improving the application and drying property of the coating liquid at the time of the production of the film formulation or improving the antiseptic property of the coating liquid, the ethanol concentration thereof is preferably 15% or less, more preferably 10% or less.

Further, when any one of the active ingredients is a protein-based active ingredient, attention needs to be paid to its denaturation or deactivation due to thermal load at the time of the application and drying of the coating liquid. Specifically, the protein-based active ingredient is desirably added to a drug layer different from those of the other active ingredients. Further, a low-viscosity grade is desirably used as a water-soluble polymer to be used in the drug layer having added thereto the protein-based active ingredient. The use of such polymer can reduce the amount of the solvent in the coating liquid, and hence can reduce a thickness at the time of the application of the prepared liquid. Thus, the drying time of the liquid is shortened, and hence the thermal load can be alleviated.

When the film formulation of the present invention is administered into an oral cavity, the formulation is disintegrated or dissolved by saliva in the oral cavity. The drug of the formulation and a thickener, which is a base component, are present together, and hence easily adhere well to the surface of the oral mucosa. In contrast, when the drug is administered as a liquid formulation, the drug is liable to be diluted and liable to flow in the oral cavity because the liquid formulation includes a water-soluble substance. Accordingly, higher drug efficacy is expected from a case in which the drug is administered as a film formulation than a case in which the drug is administered as a liquid formulation or the like.

The film formulation of the present invention is preferably used by being applied to the oral mucosa of a non-human animal.

As described above, in pet animals such as dogs and cats, the number of oral diseases, such as periodontal disease, gingivitis, and dental caries, has been increasing in recent years, and hence simple means for administration has been desired in their prevention and treatment. When the film formulation of the present invention is applied to the oral mucosa of such animal, in particular, its gums, the formulation quickly adheres to the mucosa, and its film quickly dissolves to release its drug into the oral cavity. Thus, the drug is retained in the oral cavity over a long time period, and hence oral diseases in animal can be prevented and treated.

In addition, the preferred application site of the film formulation of the present invention is a buccal side gum, which a tongue or the like does not easily reach, out of the gums. Accordingly, the film formulation of the present invention preferably has such a size as to be capable of being applied to the gums.

Next, the present invention is described in more detail by way of Examples, but the present invention is not limited to these Examples.

Each of formulations in Table 1 uses a highly mucoadhesive, easily water-soluble polymer, and may be referred to as "adhesive (AD) film" or "ADF type."

Each of formulations in Table 2 uses only an easily water-soluble polymer, and may be referred to as "orally disintegrating (OD) film" or "ODF type."

### [Formulation Production Example]

**Table 1**

| Formulations of Examples (percentage of each layer) | | | |
|---|---|---|---|
| Layer name | Component name | Example 1 | Example 2 |
| Coating layer | Pullulan | 79.5 | 79.5 |
| | D-Sorbitol | 20 | 20 |
| | Sucrose fatty acid ester | 0.5 | 0.5 |
| | Subtotal (solid content) | 100 | 100 |
| | Preparation solvent | Water | Water |
| Drug layer I | HPMC (TC-5R) | 48.1 | 48.1 |
| | Macrogol 400 | 9.9 | 9.9 |
| | Lactoferrin | 27.1 | 27.1 |
| | Lactoperoxidase | 3.4 | 3.4 |
| | Erythritol | 11.5 | 11.5 |
| | Subtotal (solid content) | 100 | 100 |
| | Preparation solvent | Water | Water |
| Drug layer II | HPC-M | 49.1 | 40 |
| | PVP K-90 | 17 | 13.8 |
| | Macrogol 400 | 9.4 | 7.7 |
| | Lactic acid bacteria (dead) | 9.4 | 7.7 |
| | Bay leaf extract | 9.4 | 0 |
| | Complex extract of licorice and balloon flower roots | 0 | 30.8 |
| | Licorice extract | 5.7 | 0 |
| | Subtotal (solid content) | 100 | 100 |
| | Preparation solvent | Ethanol | Ethanol |
| Mass ratio of each layer in film formulation | Coating layer | 0.15 to 0.45 | 0.15 to 0.45 |
| | Drug layer I | 1 | 1 |
| | Drug layer II | 1 to 3 | 1.5 to 3.5 |

A film formulation having the configuration "(coating layer)/(drug layer I)/(drug layer II)/(drug layer I)/(coating Layer)" was prepared by the following method. Table 1 shows the formulation (%) of each of the coating layer, the drug layer I, and the drug layer II.

The components shown in Table 1 were added to water (an appropriate amount) and mixed by stirring to prepare a coating layer coating liquid. The components shown in Table 1 were added to water (an appropriate amount) and mixed by stirring to prepare a drug layer-I coating liquid. The components shown in Table 1 were added to ethanol (an appropriate amount) and mixed by stirring to prepare a drug layer-II coating liquid.

A multi-layer film having the target configuration was obtained by combining the method 1 to the method 3 described above. Further, the film was cut into a predetermined shape to provide film formulations. The resultant film formulations were packaged one by one in aluminum pouches. The thicknesses (application clearances) of the respective coating liquids at the time of their application were adjusted so that the mass ratios of the coating layer, the drug layer I, and the drug layer II in one film formulation were the values in Table 1.

**Table 2**

| Formulations of Examples (percentage of each layer) | | | |
|---|---|---|---|
| Layer name | Component name | Example 3 | Example 4 |
| Drug layer I | HPMC (TC-5R) | 43.5 | 43.5 |
| | Macrogol 400 | 8.7 | 8.7 |
| | Lactoferrin | 30.9 | 30.9 |
| | Lactoperoxidase | 3.9 | 3.9 |
| | Erythritol | 13 | 13 |
| | Subtotal (solid content) | 100 | 100 |
| | Preparation solvent | Water | Water |
| Drug layer II | HPC-SSL | 55.6 | 53.8 |
| | Macrogol 400 | 8.3 | 7.7 |
| | Lactic acid bacteria (dead cells) | 13.9 | 7.7 |
| | Complex extract of licorice and balloon flower roots | - | 30.8 |
| | Bay leaf extract | 13.9 | - |
| | Licorice extract | 8.3 | - |
| | Subtotal (solid content) | 100 | 100 |
| | Preparation solvent | Ethanol | Ethanol |
| Mass ratio of each layer in film formulation | Drug layer I | 1 | 1 |
| | Drug layer II | 0.5 to 2.5 | 0.8 to 3.8 |

A film formulation having the configuration "(drug layer I)/(drug layer II)/(drug layer I)" was prepared by the following method. Table 2 shows the formulation (%) of each of the drug layer I and the drug layer II.

Drug layer-I coating liquid: The components shown in Table 1 were added to water (an appropriate amount) and mixed by stirring to prepare a drug layer-I coating liquid.

Drug layer-II coating liquid: The components shown in Table 2 were added to ethanol (an appropriate amount) and mixed by stirring to prepare a drug layer-II coating liquid. A multi-layer film having the target configuration was obtained by applying the above-mentioned method 1 to method 3 correspondingly. Further, the film was cut into a predetermined shape to provide film formulations. The resultant film formulations were packaged one by one in aluminum pouches.

The thicknesses (application clearances) of the respective coating liquids at the time of their application were adjusted so that the mass ratios of the drug layer I and the drug layer II in one film formulation were the values in Table 3.

### [Animal Test]

### (1) Test Formulations (Film Formulations)

The following three kinds of film formulations were used.

### ·Adhesive (AD) Film:

The formulation of Example 1 cut into an elliptical shape measuring 10.6 mm in minor axis by 21.2 mm in major axis

Formulation area: 1.77 cm², formulation mass: 43.60 mg

### · Adhesive (AD) (Small) Film

The formulation of Example 1 cut into an elliptical shape measuring 7.5 mm in minor axis by 15 mm in major axis

Formulation area: 0.88 cm², formulation mass: 21.80 mg

### · Orally Disintegrating (OD) Film

The formulation of Example 3 cut into a rectangle shape measuring 14 mm in short side by 20 mm in long side

Formulation area: 2.8 cm², formulation mass: 59.00 mg

### (2) Experimental Animals

Twelve dogs each suffering from gingivitis or periodontal disease were used as test animals.

The test animals were divided into an AD film group, an AD (small) film group, and an OD film group. The grouping of the test animals was performed so that the degrees of gingivitis or periodontal disease in the respective groups were the same on the basis of a gingivitis score evaluation, a mouth odor sensory test, and a periodontal bacteria evaluation.

### (3) Gingivitis Score Evaluation

The degree of the inflammation of gingivitis was evaluated in accordance with the following table. A gingivitis score was evaluated at each of the gum sites of the following teeth.

Maxilla: a third incisor, a canine, a second premolar, a third premolar, a fourth premolar, and a first molar

Mandible: a canine, a second premolar, a third premolar, a fourth premolar, and a first molar

That is, the evaluation was performed at each of the 11 sites, and the total score of the respective sites was defined as an evaluation result. For example, when the scores of all the sites are 3, the evaluation result is 33 (3×11). The degree of the inflammation was judged a total of five times: Day 1, Day 5, Day 9, Day 16, and Day 29. Day 1 is the first day of the test, and is a day before the performance of first administration. Day 29 is the next day of the day of the performance of the last administration (28th administration).

**Table 3**

| Score | Judgment criteria | Gingival margin coloration | |
|---|---|---|---|
| 0 | No inflammation is observed at a gingival margin. | PI-6 | 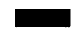 |
| 0.5 | Slight inflammation is observed at the gingival margin. | PI-3 | 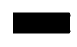 |
| 1 | Clear inflammation is observed at the gingival margin. | PI-8 | 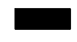 |
| 2 | Gums bleed when a probe is inserted. | PI-8 | 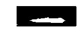 |
| 3 | The gums bleed spontaneously. | PI-8 | 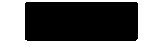 |

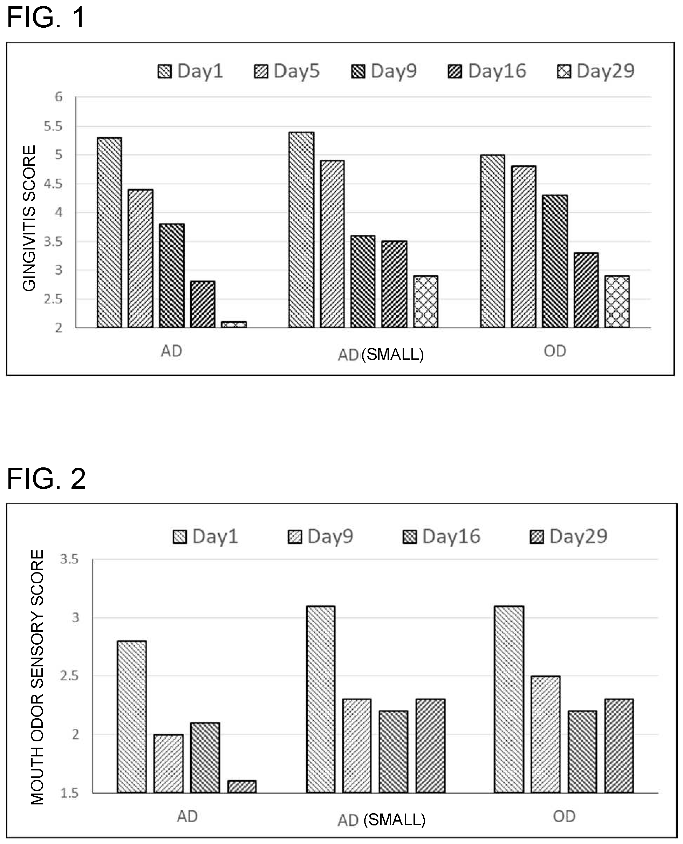

For the score evaluation of periodontitis, the color tones PI-3, PI-6, and PI-8 of "New Color Scheme Card 199a" (Japan Color Enterprise Co., Ltd.) were referenced. The evaluation results are shown in FIG. 1. FIG. 1 shows that the film formulation of the present invention exhibits preventing and treating effects on periodontitis.

### (4) Mouth Odor Sensory Test

The mouth odors of the test animals were judged by four panelists. The judgment was performed in accordance with scores in the following table from the viewpoints of an "odor intensity" and an "odor type."

The judgment was performed a total of four times: Day 1, Day 9, Day 16, and Day 29. Day 1 is the first day of the test, and is a day before the performance of the first administration. Day 29 is the next day of the day of the performance of the last administration (28th administration).

**Table 4**

| Odor intensity | |
|---|---|
| Score | Judgment criteria |
| 0 | The mouth odor is not felt at all as an odor (Odorless). |
| 1 | It is found that an odor is different from a room odor or an animal body odor, but the type of the odor cannot be specifically expressed. |
| 2 | The type of the odor can be slightly identified. The type of the odor can barely be expressed by careful sniffing. |
| 3 | The odor can be clearly identified. |
| 4 | The odor can be clearly identified, and a strong odor is felt. |

**Table 5**

| Odor type | |
|---|---|
| Score | Judgment criteria |
| A | Putrefactive odor <<sulfur compound-based>> (odor related to the degree of periodontal disease) |
| B | Slightly fishy odor (odor related to the amount of saliva secretion) |

### · Specific Approach to Test

For example, data on the mouth odor evaluation of the AD group on Day 1 was totaled as describe below.

Panelist A judged that the intensity of the mouth odor of the dog with individual number No. 1 to be 2, and judged the type of the felt odor to be B. Accordingly, a check "○" was placed in each of the boxes "Odor intensity: 2" and "Odor type: B." Panelists B to D also performed the evaluation in the same procedure. Next, the judged values of the dog with individual number No. 1 were defined as follows: the average value of the four panelists was adopted as its odor intensity, and the total of the checks for each odor was adopted as its odor type. The dogs No. 2 to 4 were judged similarly (only their judged values are listed). The final evaluation values of the AD group on Day 1 were defined as follows: the average of the judged values of the respective dogs was adopted as its odor intensity, and the total of the checks for each odor was adopted as its odor type.

**Table 6**

| Animal No. | Odor intensity | Odor type | |
|---|---|---|---|
| | | A | B |
| 1 | 1.75 | 1 | 1 |
| 2 | 3.50 | 2 | 1 |
| 3 | 3.00 | 3 | 0 |
| 4 | 2.75 | 0 | 0 |
| Evaluation value | 2.75 | 6 | 2 |

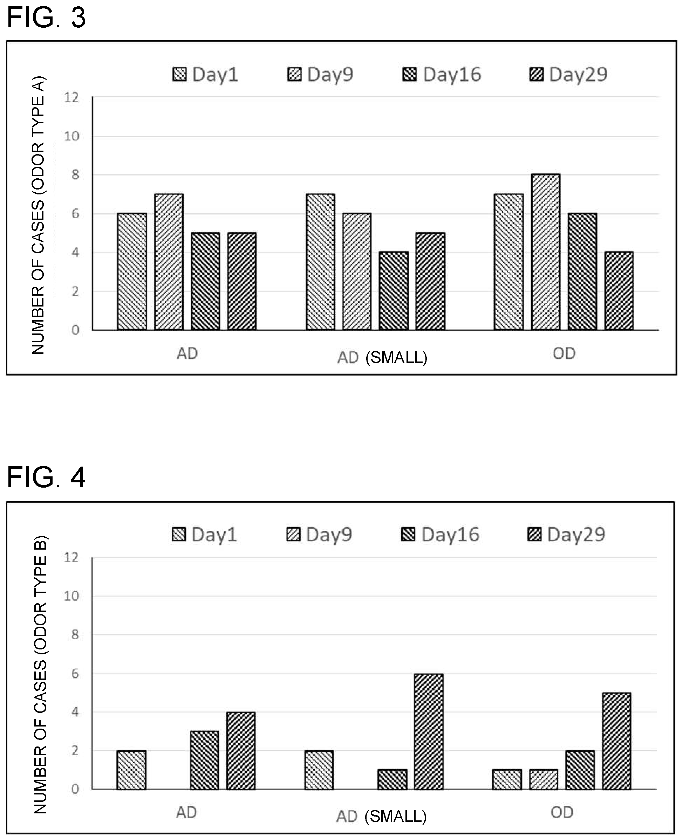

The judgment was performed on each group by the same approach, and the final evaluation results are shown in FIG. 2 to FIG. 4. When the odor intensity was judged to be 0 or 1, the odor type was not checked. FIG. 2 to FIG. 4 showed that the film formulation of the present invention exhibited a mouth odor prevention effect, in particular, reduced an odor originating from periodontal disease, and increased an original odor originating from saliva, and hence an oral cavity environment such as a moist condition in an oral cavity was improved.

### (5) Periodontal Bacteria Evaluation

A periodontal bacteria evaluation was performed by using the following two kinds of measurement kits: OraStrip (DS Pharma Animal Health Co., Ltd.) and ADplit (Kyoritsu Seiyaku Corporation).

Measurement sites ranged from the canine (C) to the fourth premolar (P4), and the evaluation was performed at four sites in the left and right upper and lower jaws. The average value of concentrations (scores) at the four sites was defined as a measurement result.

The OraStrip is a commercially available measurement kit for measuring the concentration of "thiol" serving as a metabolite of anaerobic bacteria in an oral cavity. The ADplit is a commercially available kit for measuring the concentration of an enzyme "N-benzoyl-DL-arginyl peptidase," which is specifically produced from periodontal pathogens in a dog. In each of both the kits, the measurement result is obtained as a score of from 0 to 5. A larger score means that the concentration of the target component is higher, that is, the symptom of gingivitis or periodontal disease is more severe.

Judgment using the OraStrip was performed a total of four times: Day 1, Day 9, Day 16, and Day 29. Day 1 is the first day of the test, and is a day before the performance of the first administration. Day 29 is the next day of the day of the performance of the last administration (28th administration).

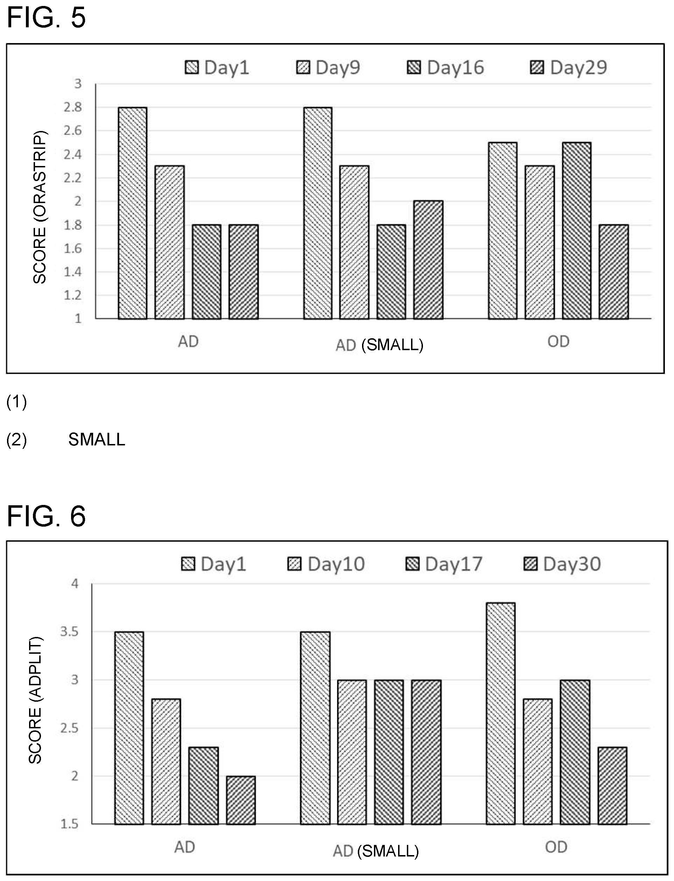

Judgment using the ADplit was performed four times: Day 1, Day 10, Day 17, and Day 30. Day 1 is the first day of the test, and is a day before the performance of the first administration. Day 30 is a day two days after the performance of the last administration (28th administration). The results of the periodontal bacteria evaluation are shown in FIG. 5 and FIG. 6. FIG. 5 and FIG. 6 show that the film formulation of the present invention suppresses the increase of periodontal bacteria in an oral cavity.

### (6) Administration of Test Formulation

### · Feeding of Food and Water

The test animals were fed once a day between 9:00 and 11:00. The test animals were allowed to freely consume water during the test.

### · Administration of Test Formulation

A predetermined film formulation was applied between 13:00 and 14:00. Before the application, it was recognized that there was no remaining food in a mouth and in a cage. When the remaining food was present, the food was removed. On the day of the evaluation of the state of gingivitis or periodontal disease, the film formulation was applied after the corresponding evaluation had been performed.

### (7) Evaluation of Dissolution Time

### · Experimental Animals

Sixteen dogs were used as test animals.

### · Evaluation Method

One of the formulations, that is, the adhesive (AD) film, the adhesive (AD) (small) film, or the orally disintegrating (OD) film was applied to the gums of each of the dogs. The presence or absence of the formulation was visually observed 15 minutes and 30 minutes after the application. A case in which even though the formulation was small, the formulation was able to be visually observed was evaluated as "present", and a case in which the formulation was not able to be visually observed was evaluated as "absent". When the result of the evaluation at 15 minutes was "absent", the evaluation at 30 minutes was not performed.

**Table 7**

| · Test results | | | |
|---|---|---|---|
| No. | Applied formulation | Presence or absence of formulation | |
| | | 15 minutes after | 30 minutes after |
| 1 | AD | Present | Absent |
| 2 | OD | Absent | - |
| 3 | AD | Absent | - |
| 4 | AD small | Present | Present |
| 5 | OD half | Absent | - |
| 6 | OD | Present | Absent |
| 7 | AD | Absent | - |
| 8 | AD small | Present | Absent |
| 9 | AD small | Absent | - |
| 10 | AD small | Absent | - |
| 11 | AD small | Present | Absent |
| 12 | AD small | Present | Absent |
| 13 | AD small | Present | Absent |
| 14 | AD small | Absent | - |
| 15 | AD small | Present | Absent |
| 16 | AD small | Present | Absent |

**Table 8**

| Applied formulation | Number of animals applied | Number of individuals with formulation | | Rate after 15 minutes |
|---|---|---|---|---|
| | | 15 minutes after | 30 minutes after | |
| ADF type | 13 | 8 | 1 | 61.5% |
| ODF type | 3 | 1 | 0 | 33.3% |

With regard to the extent to which a formulation dissolved, most of the ADF type formulations dissolved within 15 minutes to 30 minutes, and most of the ODF type formulations dissolved within 15 minutes. The residual ratios of the formulations after 15 minutes were as follows: the residual ratio of the ADF type formulations was 61.5% (8/13), and that of the ODF type formulations was 33.3% (1/3).

As observed in FIG. 1 to FIG. 6, the therapeutic effect of the ADF type formulation tends to be higher. This is considered to be because the ADF type formulation remains longer in an oral cavity (on gums), and hence its active ingredients remain in the oral cavity longer.

However, in a short-headed dog or cat, its gums are small, and hence it may be difficult to apply the formulation to the inside of its oral cavity. In addition, depending on, for example, the personality of each individual, when the formulation is present on its gums, the animal may try to remove the formulation by using its tongue.

In such case, the ODF type formulation may provide a higher therapeutic effect because the formulation dissolves in a short time period and its active ingredients remain on the surface of the gums in a viscous state.

## Claims

1. An animal oral cavity-adhesive film formulation, comprising at least one film layer containing a prophylactic or therapeutic agent for an animal disease and a highly mucoadhesive, easily water-soluble polymer.

2. The film formulation according to claim 1, wherein the film formulation is an animal oral cavity-adhesive film formulation comprising two or more film layers in which an outermost film layer is a film layer containing the prophylactic or therapeutic agent for an animal disease and the highly mucoadhesive, easily water-soluble polymer, or a film layer containing the highly mucoadhesive, easily water-soluble polymer.

3. The film formulation according to claim 1, wherein the highly mucoadhesive, easily water-soluble polymer is one or two or more kinds selected from pullulan, polyvinylpyrrolidone, polyacrylic acid, and carboxyvinyl polymer.

4. The film formulation according to any one of claims 1 to 3, wherein the film formulation has a thickness of from 50 µm to 1,000 µm.
